# EUROPEAN PATENT APPLICATION

(11) **EP 1 758 038 A1**
(43) Date of publication of application: **28.02.2007**
(21) Application number: 05018189.0
(22) Date of filing: 22.08.2005
(51) Int. Cl.: G06F 19/00

(54) **Computer-implemented method, system, computer program product and data structure for drawing up a nutrition plan**

(71) Applicant: InterComponentWare AG, 69190 Walldorf (DE)
(72) Inventor: Elitok, Ercan, 89073 Ulm (DE)
(74) Representative: Müller-Boré & Partner Patentanwälte

(57) **Abstract**

The present invention relates to a computer-based method, system, computer program product and data structure for drawing up a nutrition plan for at least one patient and/or a group of patients by a user comprising:
- a step (S1) of providing a food database (100) defining the composition of a plurality of food products composed of at least part of its food ingredients;
- a step (S2) of receiving an ingredient grading input by the user for establishing at least one grading for at least one food ingredient;
- a step (S3) of assigning the at least one grading to a food ingredient grading scheme (110);
- a step (S4, S4') of applying the food ingredient grading scheme (110) to at least part of the food database (100) for achieving a grading for at least part of the food products; and
- a step (S5, S5') of assigning the food ingredient grading scheme (110) and/or at least part of the graded food products (122) to the at least one patient and/or the group of patients.

## Description

The present invention refers to a computer-implemented method and system as well as computer program product and data structure for drawing up or generating a nutrition plan.

Generally, a nutrition plan defines instructions for a subject and in particular for a patient indicating which kind and which amount of food he should eat and what he should drink or what he should avoid to eat or drink in order to achieve a certain goal such as loosing weight or healing a disease. The instructions of a nutrition plan may comprise precise indications of an exactly defined meal composition or more general suggestions for eating or avoiding certain categories of food such as vegetables, fruits, meat, etc.

For both aesthetic and medical reasons the interest in such nutrition plans increases. Accordingly, extensive and detailed lists have been elaborated indicating which foodstuff is suitable in a more or less specified personal situation or which combination of foodstuff should be avoided. Some of these instruction and suggestions also have proven their positive effects in actual experiences of individual subjects and in particular in the experiences of patients. However, one particular nutrition plan is not necessarily suitable for all patient, even when trying to achieve the same goal, e.g. loosing weight.

There have been various approaches for providing and adapting a nutrition plan to the particular requirements of different patients. Some of these systems define the nutrition plan in terms of food categories, such as fruits and meat, for example, or they merely distinguish between animal products and vegetarian products. These systems are quite easy to memorise and to apply. They are, however, not very specific to the details of personal requirements. Other systems, on the other hand, are based on extensive and detailed lists for exactly defining which foodstuff is suitable for a detailed particular personal situation. These systems, however, are rather complicated to apply and relatively inflexible for the respective patient.

It is the object of the present invention to provide a computer-based method, a system, a computer program product, and a data structure for drawing up a nutrition plan that provides an improved flexibility for adaptation to personal nutrition requirements of a patient while making the operation through the patient easier.

This object is solved according to the invention by the features of the independent claims. Preferred embodiments of the invention are subject of the dependent claims.

According to the present invention, there is provided a computer-based method of drawing up or generating a nutrition plan or scheme for at least one patient and/or a group of patients and/or a team by a user, such as a nutritionist and/or a patient comprising:
- a step of providing a food database defining the composition of a plurality of food products, such as apples, potatoes, cheese, etc., composed of at least part of its food ingredients such as vitamins, proteins, fats, sodium, iron etc.;
- a step of receiving an ingredient grading input by the user for establishing at least one grading or rating or classification or rank for at least one food ingredient;
- a step of assigning the at least one grading to a food ingredient grading scheme;
- a step of applying the food ingredient grading scheme to at least part of the food database for achieving a grading or rating or classification or rank for at least part of the food products; and
- a step of assigning the food ingredient grading scheme and/or at least part of the graded and/or rated and/or classified food products to the at least one patient and/or the group of patients.

Accordingly, the method increases the flexibility for adapting a nutrition plan to the individual requirements for a particular patient or a group of patients. In particular, a precise adaptation can be achieved by adapting and/or creating a food ingredient grading scheme, which preferably comprises up to several tens of ingredient gradings preferably less than one hundred even more preferrably less than 50 and most preferably less than 20 or even less than 10 ingredient gradings. It is obviously much easier to adapt parameters for this number of gradings than adapting parameters for hundreds, thousands or even ten thousands of gradings for graded food products.

A further advantage of the above method is particularly the high preciseness and the reliability of the adaptation of a nutrition plan to the precise requirements for the at least one patient or the group of patients, and particularly to the detailed medical requirements. As the nutrition plan is based on the gradings/ranks/rating/classification of individual food ingredients, which are the most reliable and most direct parameters for defining the nutrition requirements and/or the medical requirements, the achieved nutrition plan is very close to the actual requirements.

In this method the step of applying the food ingredient grading scheme to at least part of the food database can be applied before or after the step of assigning the food ingredient grading scheme and/or at least part of the graded food products to the at least one patient.

Preferably, the step of assigning the food ingredient grading scheme and/or at least part of the graded food products to the at least one patient comprises the steps of:
- assigning the food ingredient grading scheme and/or at least part of the graded food products to at least one group of patients and/or a team; and
- assigning the at least one patient to the group of patients and/or team.

Further, it is preferred that the step of assigning at least part of the graded food products to the at least one patient and/or the group of patients comprises the steps of:
- presenting a plurality of graded food products together with the grading of the food products;
- receiving a food product selection input by the user for achieving a selection of graded food products;
- assigning the selection of graded food products to the at least one patient.

Further, in a preferred embodiment the step of assigning the selection of graded food products to the at least one patient comprises receiving meal composition input by the user for achieving the composition of at least one meal, such as breakfast, lunch, or dinner, composed of graded food products. Preferably a plurality of meal compositions are combined and form a well-defined diet which is preferably assigned to a patient.

Preferably, the step of assigning the food ingredient grading scheme and/or the graded food products to the at least one patient and/or the group of patients comprises
- composing at least one diet program scheme comprising the food ingredient grading scheme and/or the graded food products; and
- assigning the diet program scheme to the at least one patient and/or the group of patients.

Even further is preferred if the method further comprising the steps of
- receiving a general goal reference value input for establishing at least one general goal scheme defining at least one reference value for low and/or high limits for at least one of the group comprising body weight, body mass index, blood pressure, heart rate, and total cholesterol; and/or
- receiving a nutrition goal reference value input for establishing at least one nutrition goal scheme defining at least one reference value for low and/or high limits for the intake/day for at least one of the group comprising calories, fat, protein, carbohydrates, fluoride, folic acid, iron, and iodine; and/or
- receiving physical activity prescription input for establishing at least one set of physical activity prescriptions; and
where the method further comprises
- assigning a general goal scheme and/or a nutrition goal scheme and/or a set of physical activity prescriptions to the at least one diet program scheme.

Preferably, the step of receiving an ingredient grading input comprises
- receiving an ingredient selection input by the user defining [the selection of] at least one ingredient to be graded;
- receiving a grading reference value input for achieving at least one ingredient grading reference value, which the amount for the selected food ingredient should be compared to;
- receiving a grading interval input by the user defining a plurality of grading intervals each of which defines a percentage interval relative to the reference value; and
- assigning a grade to each of the grading intervals.

The at least one ingredient grading reference value is preferably selected from the group comprising the recommended daily allowance (RDA) for the particular food ingredient, the upper safe intake level (UL) or (tolerable) upper intake limit for the food ingredient, the total weight of the food product, and the total amount of calories of the food product.

The method further preferably comprises a step of providing at least one standard reference value database defining a standard reference value for at least one type of reference values selected from the reference values for the general goals and/or the nutrition goals and/or the food ingredient grading,
wherein the steps of receiving a reference value input comprise the steps of
- receiving a reference selection input for defining and/or selecting at least one reference value type, such as the recommended daily allowance (RDA) and/or the upper intake limit (UL) and/or the percentage in weight and/or the percentage in calories and/or a low and/or a high limit for at least one of the group comprising body weight, body mass index, blood pressure, heart rate, total cholesterol, and preferably other general goals; and/or a low and/or a high limit for the intake/day for at least one of the group comprising calories, fat, protein, carbohydrates, fluoride, folic acid, iron, iodine and preferably other nutrition goal; and
- retrieving the standard reference value for the defined reference value type from the at least one standard reference value database.

Preferably, the method further comprises:
- a step of providing a reference value adaptation scheme defining for a plurality of different personal status conditions comprising age and/or sex of a patient a ratio for at least one reference value relative to the standard reference value defined for standard status conditions;
- a step of receiving an input by the user for defining personal status data of the at least one patient, where the personal status data comprise age and/or sex of the at least one patient;
- a step of applying the personal status adaptation scheme to the reference values depending on the personal data for achieving personal reference values.

In a preferred embodiment the step of receiving an ingredient grading input comprises a step of receiving a weighting input by the user which defines a weighting factor for the at least one grading of the at least one food ingredient and where the grading of the at least one food product is achieved by considering the grading for at least part of its ingredients with a relative weight defined by the respective weighting factor.

Preferably, the method further comprises the steps of:
- receiving at least one high and/or low ingredient limit input by the user for defining at least one high and/or low limit for at least one food ingredient;
- assigning the at least one received ingredient limit to at least one food ingredient filter scheme; and
- applying the food ingredient filter scheme to at least part of the food database for achieving a filtering of at least part of the food products, where the filtering distinguishes between allowable and non-allowable food products; and
wherein in the step of presenting a plurality of food products only allowable food products are presented.

Preferably, the method further comprises a step of assigning the filter scheme to the at least one patient and/or the group of patients. Further preferably, the method comprises a step of assigning the filter scheme to a diet program scheme, which preferably is a data set defining details for a diet program, and a step of assigning the diet program scheme to at least one patient and/or a group of patients.

Preferably, the food ingredient grading scheme and/or the filter scheme and/or the general goals and/or the nutrition goals are saved as a template data and are available at a later time for assigning them to other patients and/or groups of patients (teams) and/or for composing further diet program schemes.

Further, the method preferably comprises after the step of assigning the diet program scheme to the at least one patient and/or the group of patients a step of receiving an adaptation input for adapting at least one of the food ingredient grading scheme, the general goals, the nutrition goals, the filter scheme and the diet to the at least one patient and/or the group of patients.

The invention further provides a computer-based method of drawing up or generating a nutrition plan or scheme for at least one patient and/or a group of patients comprising
- presenting at least one food product, such as an apple, a potato, milk, etc.;
- retrieving at least one ingredient, such as vitamins, proteins, fats, sodium, iron, etc., of the food product from a food database;
- retrieving at least one grading or rating or classification or rank for the at least one ingredient of the food product from a food ingredient grading scheme assigned to the patient and/or the group of patients;
- grading the food product depending on the at least one grading or rating or classification or rank of the at least one of its ingredients; and
- presenting the grading or rating or classification or rank of the food product together with the food product.

Accordingly, there is provided a very user friendly, and in particular a very patient friendly method of drawing up a nutrition plan or scheme. The method provides a grading or rating or classification or rank of food products to at least one patient, where the grading is based on detailed nutrition requirements and medical requirements for the at least one patient defined in terms of ingredient gradings, which offers a very precise and reliable basis. The patient, however, does not have to care about such medical details the meaning of which most patients would not understand anyway. The method, instead, presents a grading of food products to the at least one patent, so that the patient can choose suitable food products in a very convenient way without further background knowledge about the details of his personal nutrition requirements and/or medical requirements.

Preferably, the step of retrieving at least one ingredient comprises retrieving a plurality of ingredients of the at least one food product;
where determining at least one grading for the at least one ingredient comprises
- determining at least one grading for at least part of the plurality of ingredients and
- determining and/or setting a weighting factor of the at least one grading for each ingredient; and
where in the step of grading the at least one food product the grading for each ingredient of the food product is considered with a weight defined by the associated weighting factor.

According to the invention, there is further provided a computer-implemented system for drawing up or generating a nutrition plan or scheme for at least one patient and/or a group of patients and/of a team by a user, such as a nutritionist and/or a patient and/or a doctor, in particular performing operations according to a method of the present invention or a preferred embodiment thereof, comprising:
- food product composition storage means for storing at least one food database defining the composition of a plurality of food products, such as apples, potatoes, cheese, etc., composed of at least part of its food ingredients, such as vitamins, proteins, fats, sodium, iron etc.;
- ingredient grading input means for receiving an input by the user for establishing at least one grading or rating or classification or rank for at least one food ingredient;
- grading scheme composition means for composing at least one food ingredient grading scheme by assigning the at least one grading or rating or classification or rank for at least one food ingredient to the at least one food ingredient grading scheme;
- food product grading means for grading at least part of the food products defined in the at least one food database depending on the grading or rating or classification or rank of at least one of its ingredients according at least one food ingredient grading scheme; and
- nutrition plan composition means for assigning the food ingredient grading scheme and/or at least part of the graded food products to a nutrition plan for the at least one patient and/or the group of patients.

The system preferably comprises food product grading storing means for storing the grading of the food products.

According to the invention, there is still further provided a computer-implemented system for drawing up or generating a nutrition plan or scheme for at least one patient and/or a group of patients and/or at least one team by a user, such as a nutritionist and/or a patient and/or a doctor, in particular performing operations according to a method of the present invention or a preferred embodiment thereof, comprising:
- food product composition storage means for storing at least one food database defining the composition of a plurality of food products, such as apples, potatoes, cheese, etc., composed of at least part of its food ingredients, such as vitamins, proteins, fats, sodium, iron etc.;
- food ingredient grading storage means for storing at least one food ingredient grading scheme assigned to the at least one patient and/or the group of patients, where the at least one food ingredient grading scheme defines at least one grading or rating or classification or rank for at least one food ingredient, such as vitamins, proteins, fats, sodium, iron etc.;
- food product grading means for grading at least one food product defined in the at least one food database depending on the grading or rating or classification or rank of at least one of its ingredients according to the at least one food ingredient grading scheme; and
- food product presenting means for presenting the at least one graded food product together with its grading.

According to the invention, there is further provided a computer program product, particularly tangibly stored on a medium, comprising program code, which when loaded in a computer system causes the system to perform step according to a method according to the invention or a preferred embodiment thereof.

According to the present invention, there is still further provided a data structure preferably integrated as an element of a nutrition plan or scheme for at least one patient, and/or a group of patients comprising:
- at least one food database defining the composition of a plurality of food products, such as apples, potatoes, cheese, etc., composed of at least part of its food ingredients, such as vitamins, proteins, iron, etc.;
- at least one food ingredient grading scheme defining a grading or rating or classification or rank for at least one food ingredient;
where a link is established from the at least one graded food ingredient to the respective ingredient defined in the at least one food database as a component of at least one food product such that this link results in a grading of the at least one food product.

Preferably, the composition of the plurality of food products is defined quantitatively.

The data structure preferably further comprises
- patient database for defining personal data for at least one patient or a group of patients;
- a diet program scheme database, that defines at least one diet program scheme; and
where the data structure further comprises
- a physical activity database comprising at least one physical activity prescription scheme defining a prescription for physical activity as part of a nutrition plan; and/or
- a general goal database comprising at least one general goal scheme defining at least one reference value for low and/or high limits for at least one of the group comprising body weight, body mass index, blood pressure, heart rate, and total cholesterol; and/or
- a nutrition goal database comprising at least one nutrition goal scheme defining at least one reference value for low and/or high limits for the intake/day for at least one food ingredient; and/or
- a filter scheme database comprising at least one ingredient filter scheme defining at least one high and/or low limit for at least one food ingredient; and/or
- a diet scheme database comprising at least one diet scheme defining for at least one meal its composition of at graded food products;
where each diet program scheme defines at least one data link that assigns the at least one food ingredient grading scheme and/or physical activity scheme and/or general goal scheme and/or nutrition goal scheme and/or filter scheme and/or diet scheme to a nutrition plan for at least one patient and/or a group of patients.

Even further, the food ingredient grading scheme preferably comprises reference values relative to which the food ingredients are graded, and the data structure preferably further comprises
- a personal database for defining personal data regarding the at least one patient; and
- a reference value database comprising at least one reference value data scheme defining reference values depending on the personal data for at least the nutrition goal scheme and/or the general goal scheme and/or the food ingredient grading scheme.

These and other objects, features and advantages of the present invention will become more apparent upon reading of the following detailed description of preferred embodiments and accompanying drawings. It should be understood that even though embodiments are separately described, single features thereof may be combined to additional embodiments.
- Fig. 1: shows an exemplary system for implementing the present invention or a preferred embodiment thereof;
- Fig. 2: shows a schematic of a preferred embodiment of the present invention;
- Fig. 3: shows an excerpt from a food database according to a preferred embodiment of the present invention;
- Fig.4: shows a food ingredient grading scheme according to a preferred embodiment of the present invention;
- Fig. 5: shows a flow diagram for a method according to a preferred embodiment of the present invention;
- Fig. 6: shows an exemplary screen display of a preferred input interface for receiving food ingredient grading data according to step S2 in Figs. 2 or 5
- Fig. 7: shows a preferred screen display of an input interface for composing a food shelf in accordance with a preferred embodiment of the present invention.
- Fig. 8: shows an exemplary screen display of an input interface for receiving high and/or low limit values for food ingredients;
- Fig. 9: shows an exemplary screen display of a preferred user interface for defining a food product based diet (well-defined diet);
- Fig. 10: shows an exemplary screen display of a preferred user interface for defining a diet program scheme;
- Fig. 11: shows an excerpt from a reference value adaptation database according to a preferred embodiment of the present invention; and
- Fig. 12: shows a block diagram representing a further preferred embodiment of the present invention.

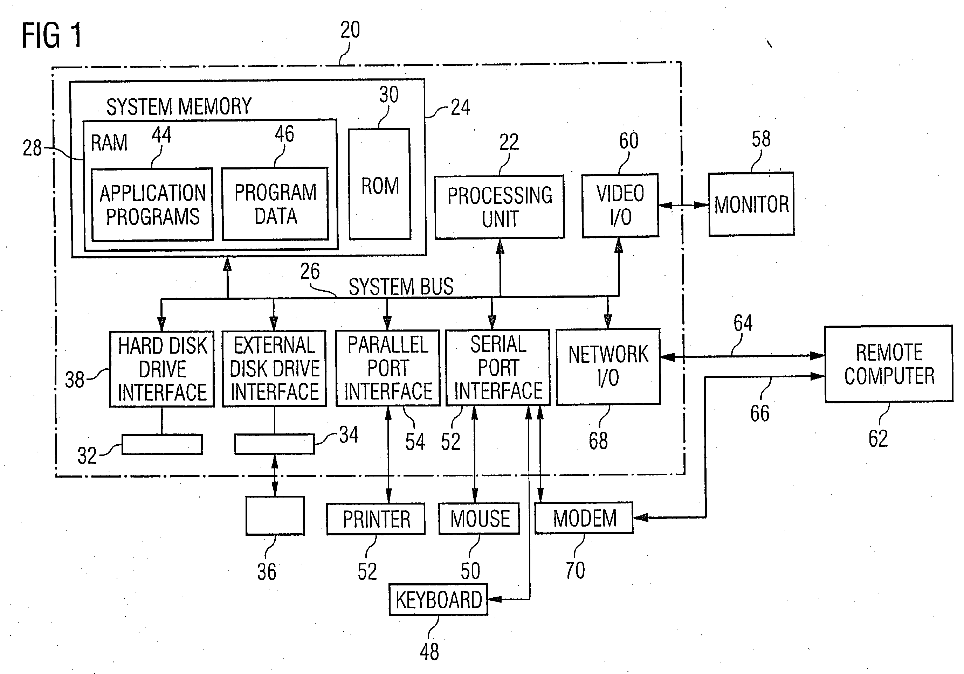

With reference to Fig. 1, an exemplary system for implementing the invention includes a general purpose computing device in the form of a conventional computing environment 20 (e.g. personal computer), including a processing unit 22, a system memory 24, and a system bus 26, that couples various system components including the system memory 24 to the processing unit 22. The processing unit 22 may perform arithmetic, logic and/or control operations by accessing system memory 24. The system memory 24 may store information and/or instructions for use in combination with processing unit 22. The system memory 24 may include volatile and non-volatile memory, such as random access memory (RAM) 28 and read only memory (ROM) 30. A basic input/output system (BIOS) containing the basic routines that helps to transfer information between elements within the personal computer 20, such as during start-up, may be stored in ROM 30. The system bus 26 may be any of several types of bus structures including a memory bus or memory controller, a peripheral bus, and a local bus using any of a variety of bus architectures.

The personal computer 20 may further include a hard disk drive 32 for reading from and writing to a hard disk (not shown), and an external disk drive 34 for reading from or writing to a removable disk 36. The removable disk may be a magnetic disk for a magnetic disk driver or an optical disk such as a CD ROM for an optical disk drive. The hard disk drive 34 and external disk drive 34 are connected to the system bus 26 by a hard disk drive interface 38 and an external disk drive interface 40, respectively. The drives and their associated computer-readable media provide nonvolatile storage of computer readable instructions, data structures, program modules and other data for the personal computer 20. The data structures may include relevant data of the implementation of the insurance claim management evaluation method, as described in more details below. The relevant data may be organized in a database, for example a relational or object database.

Although the exemplary environment described herein employs a hard disk (not shown) and an external disk 42, it should be appreciated by those skilled in the art that other types of computer readable media which can store data that is accessible by a computer, such as magnetic cassettes, flash memory cards, digital video disks, random access memories, read only memories, and the like, may also be used in the exemplary operating environment.

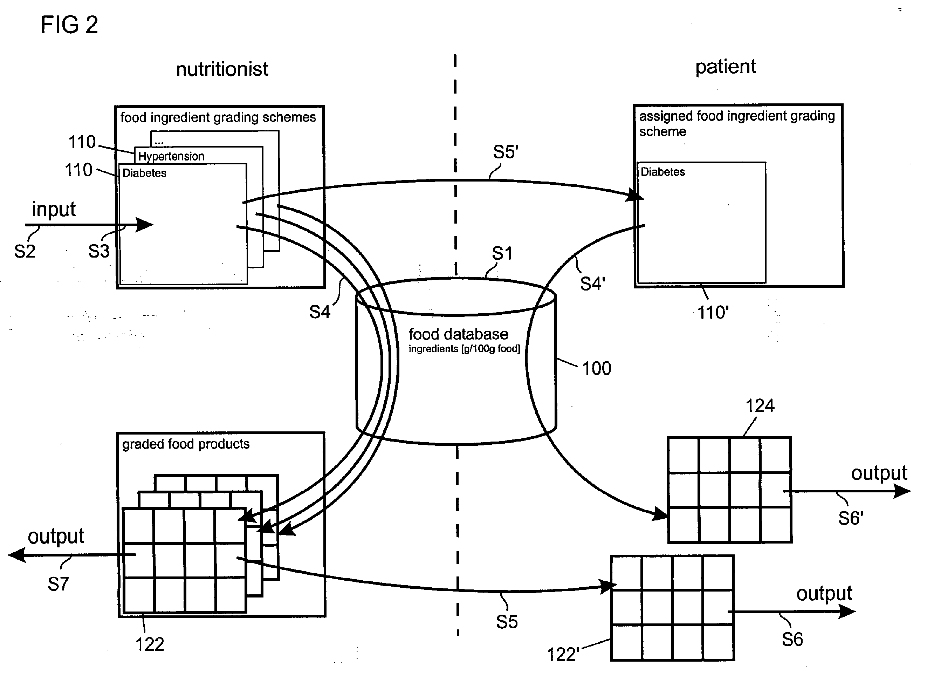

A number of program modules may be stored on the hard disk, external disk 42, ROM 30 or RAM 28, including an operating system (not shown), one or more application programs 44, other program modules (not shown), and program data 46. The application programs may include at least a part of the functionality as detailed in Figs. 2 to 11.

A user may enter commands and information, as discussed below, into the personal computer 20 through input devices such as keyboard 48 and mouse 50. Other input devices (not shown) may include a microphone (or other sensors), joystick, game pad, scanner, or the like. These and other input devices may be connected to the processing unit 22 through a serial port interface 52 that is coupled to the system bus 26, or may be collected by other interfaces, such as a parallel port interface 54, game port or a universal serial bus (USB). Further, information may be printed using printer 56. The printer 56, and other parallel input/output devices may be connected to the processing unit 22 through parallel port interface 54. A monitor 58 or other type of display device is also connected to the system bus 26 via an interface, such as a video input/output 60. In addition to the monitor, computing environment 20 may include other peripheral output devices (not shown), such as speakers or other audible output.

The computing environment 20 may communicate with other electronic devices such as a computer, telephone (wired or wireless), personal digital assistant, television, or the like. To communicate, the computer environment 20 may operate in a networked environment using connections to one or more electronic devices. Fig. 1 depicts the computer environment networked with remote computer 62. The remote computer 62 may be another computing environment such as a server, a router, a network PC, a peer device or other common network node, and may include many or all of the elements described above relative to the computing environment 20. The logical connections depicted in Fig. 1 include a local area network (LAN) 64 and a wide area network (WAN) 66. Such networking environments are commonplace in offices, enterprise-wide computer networks, intranets and the Internet.

When used in a LAN networking environment, the computing environment 20 may be connected to the LAN 64 through a network I/O 68. When used in a WAN networking environment, the computing environment 20 may include a modem 70 or other means for establishing communications over the WAN 66. The modem 70, which may be internal or external to computing environment 20, is connected to the system bus 26 via the serial port interface 52. In a networked environment, program modules depicted relative to the computing environment 20, or portions thereof, may be stored in a remote memory storage device resident on or accessible to remote computer 62. Furthermore other data relevant to the application of the insurance claim management evaluation method (described in more detail further below) may be resident on or accessible via the remote computer 62. The data may be stored for example in an object or a relation database. It will be appreciated that the network connections shown are exemplary and other means of establishing a communications link between the electronic devices may be used.

The above-described computing system is only one example of the type of computing system that may be used to implement the method, system, computer program product and/or data structure for (automatically) drawing up or generating a nutrition plan. Generally, a nutrition plan includes instructions for a subject, particularly for a patient or other person, indicating which kind and which amount of food he should eat or consume and/or what he should drink and/or what food and/or drink he should avoid to eat or drink in order to achieve a certain goal such as loosing weight or healing (or not enhancing) and/or avoiding a decease. The instructions of a nutrition plan may comprise precise indications of an exactly defined meal composition (e.g. a composition of a breakfast, lunch and/or dinner) and/or more general suggestions for eating and/or avoiding certain categories of specific (predetermined or predeterminable) food products and/or specific (predetermined or predeterminable) food categories (such as vegetables, fruits, meat, etc.) and/or subcategories (such as dark or light meat, low-fat content or high fat content cheese, etc.) and/or more general suggestions for eating and/or avoiding certain ingredients and/or group of ingredients. Preferably, a nutrition plan is directed to a scheme, program, and/or a method worked out beforehand for the accomplishment of the process by which the organism ingests, digests, absorbs, transports and/or utilizes nutrients. This process enables the normal functioning, growth and maintenance and helps to maintain the balance between health and disease. Also included is the idea of an optimal balance of nutrients and whole foods, to enable the optimal performance of the body.

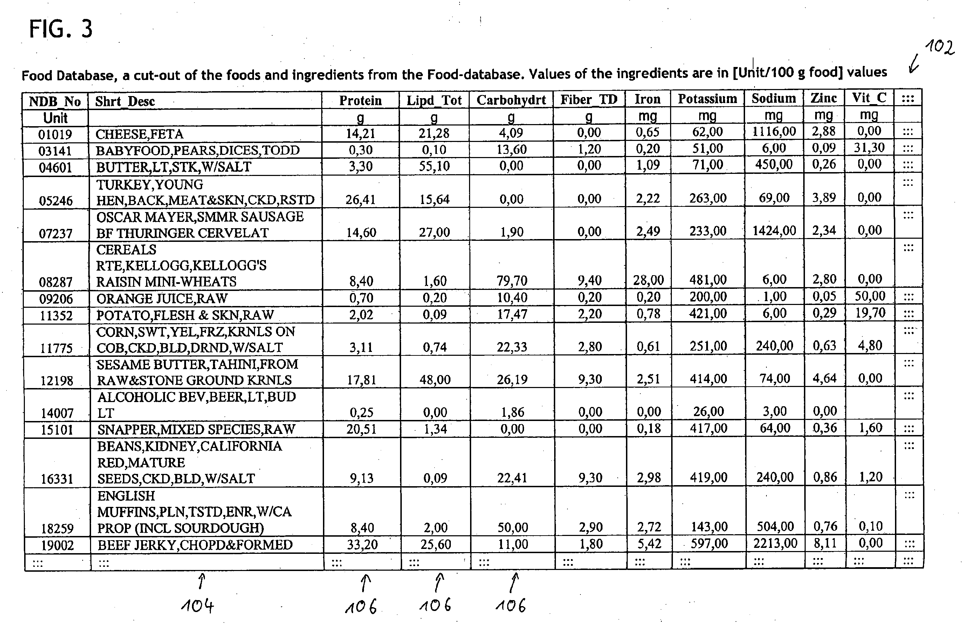

In a computer based method according to a preferred embodiment of the present invention as shown in the schematic of figure 2, in a first step S1 a food database 100 is provided, which defines or includes a composition of a plurality of food products composed of or comprising at least part of its ingredients. Preferably, the food database 100 lists the amount of each ingredients included in a specified (predetermined or predeterminable) amount (e.g. about 100 g) of the food product. Figure 3 shows a food database, a cut-out of the foods and/or ingredients from the food-database, wherein values of the ingredients are given in g/100 g food and mg/100 g food, respectively. In the shown example of an excerpt from a table 102 forming the basis for a preferred food database 100 that may be used in the method for drawing up the nutrition plan. In a food product column 104 a plurality of food products (such as their names) are listed, where each line in the table 102 represents one particular food product such as feta cheese or a particular type of cereals made by a specific producer (such as Kellogg's), for example. Among a plurality of food ingredient columns 106 each column represents or contains a particular food ingredient, such as protein, certain fat acids, carbohydrate, iron, or vitamins, for example. The food database 100, which is preferably based on such a table 102, is preferably provided by saving the data contained in table 102 in food database storage means, such as a hard disk drive 32, an external disk drive 34, or a removable disk 36 of an exemplary computer system as shown in figure 1. Preferably, the food database is also accessible to a remote computer 62 via a (mono-, bi- and/or multidirectional) network, such as the internet.

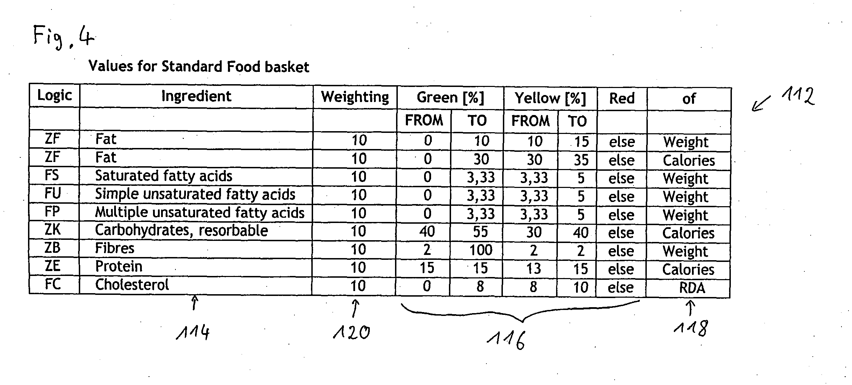

In the second step S2 of the preferred method shown in figure 2 an input is preferably received from a nutritionist (as a preferred user) who inputs data defining a specific (predetermined or predeterminable) grading or rating or classification or rank for at least one food ingredient. This grading or rating or classification or rank may be based on nutrition requirements in connection with a particular disease. For example, patients suffering from diabetes should avoid the intake of a large amount of carbohydrates. Other diseases may require a particular high or low content of vitamins and/or sodium, for example. According to these requirements, the nutritionist as the preferred user may define a certain range of the relative amount of a particular ingredient as being preferable, while other percentages of the food ingredient in a food product may be graded as less preferable as being either too low or too high. Preferably, the most preferable percentage of the particular ingredient receives (or is rated with) a high grade or rate, while less preferable amounts of the ingredient in a food product is rated or graded with a lower grade. In a further step S3 the inputted grading data are assigned to at least one food ingredient grading scheme 110. Figure 4 shows an excerpt from an exemplary table 112 forming the basis for a preferred food ingredient grading scheme 110. In a food ingredient column 114 a plurality of food ingredients are listed, where each line represents a particular food ingredient, such as fat, saturated fatty acids, protein, etc., which is contained in the respective food. In grading columns 116 percentage intervals are defined or included for each food ingredient and assigned to a particular grading or rating or classification or rank. A reference column 118 defines or includes a reference value and/or the type of a reference value relative to which the percentage of the particular food ingredient is measured. Referring to the first line defining a grading or rating or classification or rank for fat, for example, 3 percentage intervals are defined, where the first interval extends from 0 to (less than) 10%, the second in 12 extends from 10% to 15% and the third interval covers the rest. The first interval is assigned to the grade or rating or classification or rank "green" which is most preferable and preferably means "good". The second interval is assigned to the grade or rating or classification or rank "yellow", which is less preferable and represents the grading "neutral". Finally, the third interval is assigned to the grade or rating or classification or rank "red", which is least preferable and means "bad". The reference type in this line is defined as "weight", which defines that the grading intervals have to be calculated as percentage intervals with respect to the weight of the food product. Accordingly, a content of 0 to 10 g (or less than 10 g) fat in 100 g of a food product is regarded as good, a content of 10 g to 15 g fat in 100 g of a food product is grated as neutral and a even higher fat content is graded as bad. Percentages that fold exactly on the boundary values, which in the present case are 10% and 15%, are preferably assigned to the worse grade. The present example shows a second grade or rating or classification or rank for the food ingredient fat in the next line of the table 112. In this second grade or rating or classification or rank the percentage intervals have to be evaluated with respect to the total calories of a food product. Accordingly, an amount of fat that carries 0 to 30% of the total calories of a food product is regarded as being good, and so on. Finally, the last line in the exemplary table 112 represents a grading or rating or classification or rank for cholesterol, where the reference value type is the recommended daily allowance (RDA). In this grading the grading the percentage intervals are preferably related to a specified (predetermined or predeterminable) amount or weight (e.g. about 100 g) of a food product. Accordingly, the total cholesterol content in 100 g of a food product covering 0 to 8% of the recommended daily allowance (RDA) is graded as good. A cholesterol content in 100 g of a food product that covers more than 10% of the recommended daily allowance (RDA) is graded as bad, while the rest is graded neutral. Finally, the table 112 contains a weighting column 120 that's defines a relative weighting of the individual gradings or rating or classification or rank with respect to each other. Preferably, a plurality of food ingredient grading schemes 110 are created by the nutritionist and saved in storage means as templates for the later use.

According to the preferred embodiment shown in figure 2, in a next step the pre-defined food ingredient grading scheme 110 may either be applied to the food database 100 in a step S4 or it may be assigned or associated to a patient in a step S5'. While in the first case preferably the nutritionist as the preferred user applies at least one food ingredient grading scheme 110 to the food database 100 for achieving a plurality of graded food products 122 in step S4, in the second case the food ingredient grading scheme 110' assigned to the patient is applied to the food database 100 in step S4' on the patient's side for achieving a plurality of graded food products 124. After the at least one food ingredient grading scheme 110 is applied to the food database 100 in step S4 preferably a selection of the achieved graded food products 122 are assigned or associated to the patient in step S5. This selection 122' of assigned or associated graded food products is not necessarily identical to the food products 124 achieved from applying the assigned food ingredient grading scheme 110' to the food database 100 in step S4', as the step S5 of assigning graded food products 122 to the patient may comprise a step of selecting one or more particular food products. The grading or rating or classification or rank for identical food products, however, is preferably substantially identical, if both groups of graded food products of based on the same food ingredient grading scheme 110. Finally, in the steps S6, S6' and/or S7 to grading of the food products may be presented to the patient and/or the nutritionist, respectively.

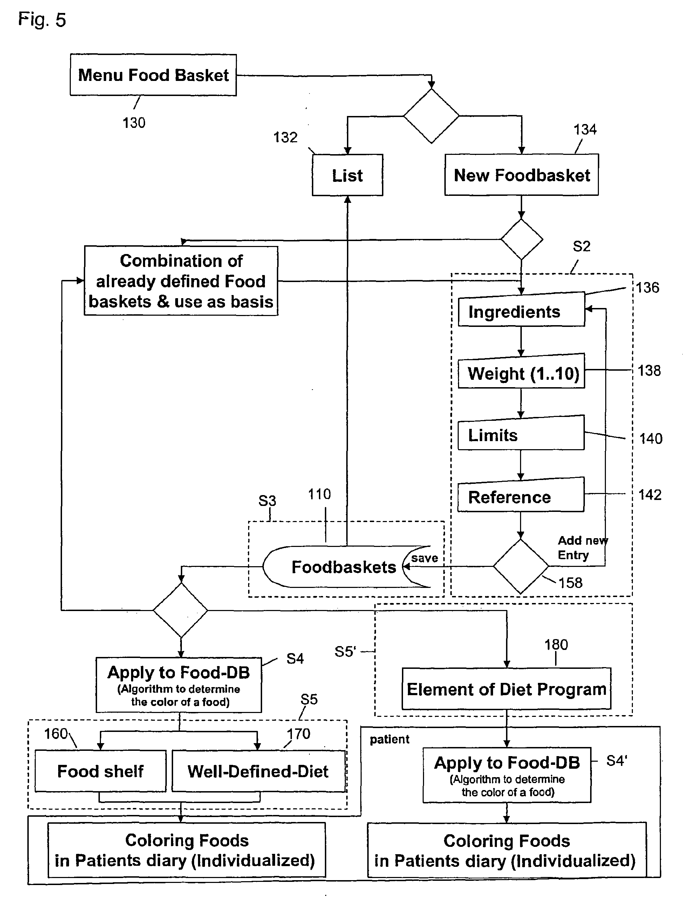

In a computer-based method according to a further preferred embodiment of the present invention as shown in the flow diagram of Fig. 5, a user enters a menu 130 for organizing and/or editing food ingredient grading schemes 110, which are also called food-baskets in the following. Directly after entering the "Menu Food Basket" 130 the user decides whether to enter a list 132 of pre-defined food ingredient grading schemes 110 or to create a new food ingredient grading scheme 110 (a new food basket). When the system receives an input 134 by the user for creating a new food ingredient grading scheme the system offers the user the choice to either create the new food ingredient grading scheme 110 based on at least one already defined food-basket or to create a totally new food basket.

Regardless of the made choice, in the following step S2 the system receives an ingredient grading input by the user for establishing at least one grading or rating or classification or rank for at least one food ingredient. In the shown preferred embodiment the step S2 of receiving an ingredient grading input comprises successive steps of receiving an input 136 for selecting and/or defining at least one particular (predetermined or predeterminable) food ingredient, a step 138 of receiving a weighting factor for the at least one food ingredient grading or rating or classification or rank, a step 140 of receiving limits of grading intervals or ranges, and a step 142 of receiving a reference value relative to which the amount of the food ingredient has to be graded.

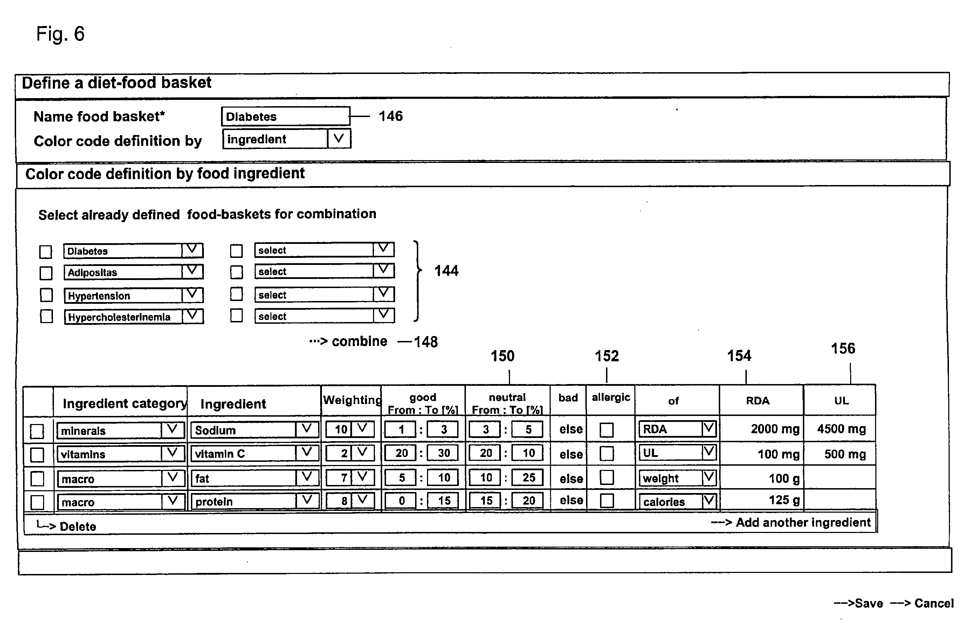

Figure 6 shows in exemplary screen display or graphical user interface (GUI) of an input interface for receiving an ingredient grading input according to step S2 as shown in figure 5. The screen display or graphical user interface (GUI) according to this preferred embodiment presents a plurality of food basket selection fields 144 for receiving an input that defines a selection of already defined food-baskets for combining the selected food-baskets to a new food basket. A name for this new food-basket(s) can be input via a name input field 146. After pressing the "combine"-button 148 a list 150 preferably presents ingredient gradings or ratings or classifications or ranks that are included in the combined previously defined food-baskets. The meanings of individual components and elements within the list 150 are preferably identical to the respective parts shown in table 112 and discussed in connection figure 4. For adapting or editing the new food-baskets the shown list 150 can be edited by the user. Particularly, the user may delete and/or add individual lines, i.e. the gradings or ratings or classifications or ranks for individual food ingredients, and/or he may edit entries within an existing line. In particular, he may change the selection of an individual ingredient such as sodium, vitamin C, fat or protein, for example, or a selection of the ingredient category such as minerals, vitamins, macro, for example, or the user may also change a weighting factor, preferably in the range from 1 to 10, a grading interval, i.e. the good, neutral, bad and irreconcilable/allergic parameters for an ingredient, or the type of the reference value, i.e. the scale type (OF), preferably from a drop down list. For each ingredient there is a default limit suggestions for good and neutral (bad is always else). These default suggestion values are preferably defined in a particular database. The user, which is preferably the therapist, can set more than one ingredient by clicking on the "Add another ingredient" button on the right bottom of the ingredients settings section.

In addition to the table 112 shown in figure 4 the presence of list 150 comprises an allergic selection column 152, where by means of flags (such as checkboxes) of food ingredient may be classified as being irreconcilable and/or indigestible and/or causing allergic reactions to the patient. The figures presented in additional columns 154 and 156 for the reference values of the recommended daily allowance (RDA) and the upper intake limit (UL) may be preferably retrieved from a standard reference value database defining these values for preferably each individual food ingredient. This standard reference value database is preferably stored on a storage means together with or independently of the food database.

Again referring to figure 5, after having entered and/or edited a grading or rating or classification or rank for a food ingredient at a branching 158 a choice can be made whether a new entry should be added, which corresponds to adding another ingredient, i.e. another line in the list 150 shown in figure 6, or the gradings or ratings or classifications or ranks of the food ingredients should be saved and assigned to the new food ingredient grading scheme 110 (food-basket) in step S3. Preferably, this new food-basket 110 is automatically added to the list 132 of already defined food-baskets. Further, this new food-basket 110 may enter the process of creating a further food-basket based on a combination of already defined food-baskets including a new one.

Moreover, the method according to the shown preferred embodiment of the present invention comprises further steps for prosecuting with the new food basket 110. This food-baskets 110 may either be directly applied to the food database 100 in the step S4 or it may be directly assigned to a patient in step S5'.

In the following an exemplary algorithm for achieving gradings or ratings or classifications or ranks (having a plurality of different levels) for food products when applying the food ingredient grading scheme 110 to the food database 100 is explained.

In the example of three grading levels for food ingredients and an additional indication for ingredients causing allergic reactions, the three levels are preferably weighted with a specific (predetermined or predeterminable) factor (e.g. values 1, 2, and 3). The allergic indication preferably does not receive a weighting. An algorithm for achieving a grading for a food product containing at least one graded ingredient preferably works like follows: 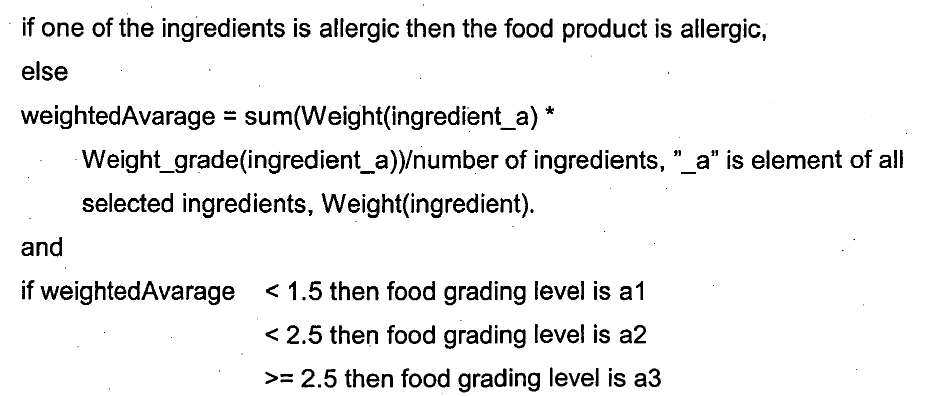 wherein for example level a1 may be 1, level a2 may be 2 and level a3 may be 3.

Accordingly, a weighted average for the grading level of a food product is preferably achieved by summing up the grading level values ai (e.g. i=1, 2 or 3) of the graded food ingredients multiplied by the weighting factor for the individual grading of the food ingredients and dividing the resulting sum by the sum of the weighting factors of all gradings of the food ingredients. Then the grading value for the food product is achieved by conventional rounding of the weighted average.

Another algorithm for combination of already defined food baskets is preferably defined as follows: If there are already defined food baskets, then the nutritionist (as the preferred user) has the possibility to combine two or more food baskets to a new one. Further, this resulting new food basket can also be modified. The predefined food baskets preferably can be selected in a drop down lists, for example, for combination. 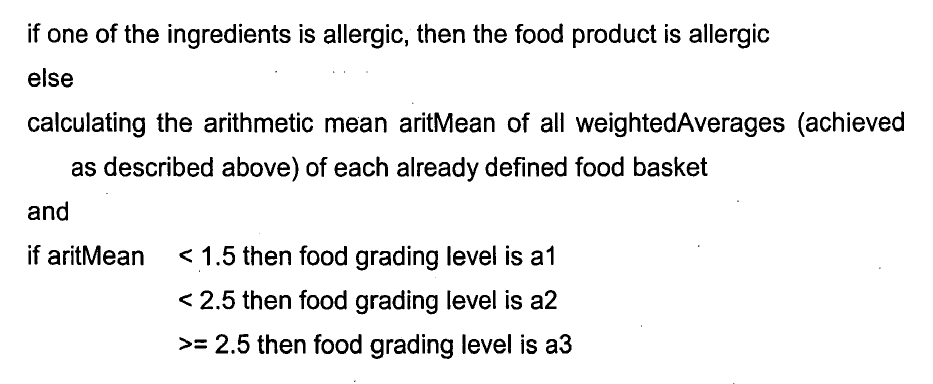 wherein for example level a1 may be 1, level a2 may be 2 and level a3 may be 3.

Firstly, addressing the first alternative of applying food-baskets 110 to the food database 100 in step S4, the graded food products achieved from the step S4 of applying the food-basket 110 to the food database 100 are preferably assigned to at least one patient and/or a group of patients and/or a team in step S5. The grading of the food products is preferably presented by means of a color code (e.g. green/yellow/red) comparable to the color code shown for the grading of the food ingredients in connection with figure 4.

In one preferred embodiment the step S5 of assigning the graded food products to the at least one patient comprises composing a food shelf 160 and assigning the food shelf 160 to the at least one patient. Preferably this food shelf 160 comprises graded food products which are preferably selected from all of the graded food products by a nutritionist depending on the grading of the food products. Accordingly, the food shelf 160 preferably is a graded subgroup of the food database. This preselection of food products makes it much easier for a user (such as a patient) to select appropriate food products as compared to the situation, where the patient himself has to choose among a huge number of available food products from the total database 100, for example.

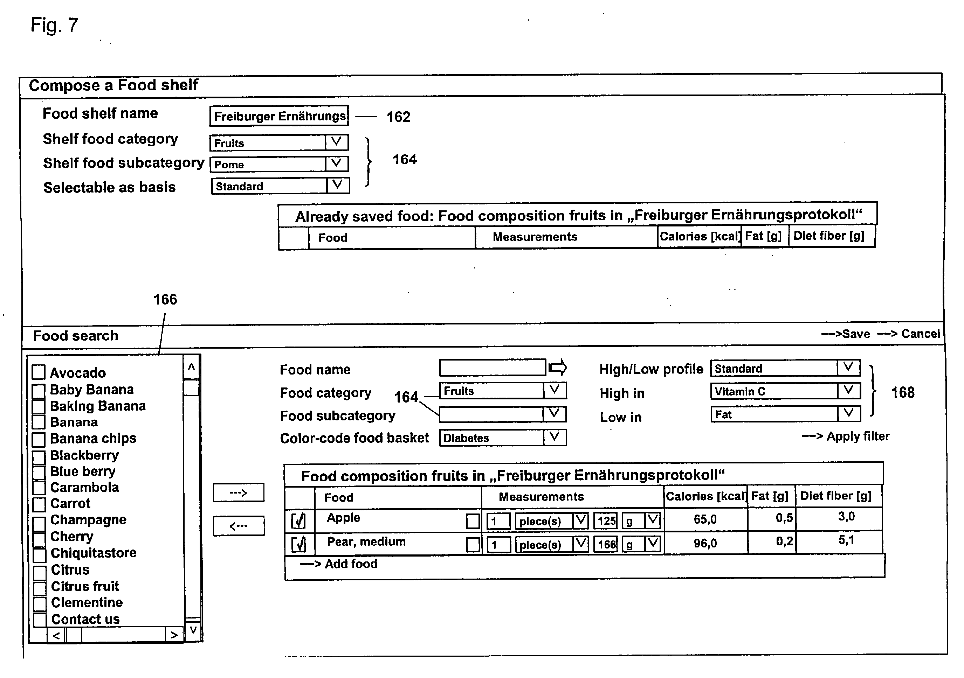

Figure 7 shows an exemplary screen display or graphical user interface (GUI) preferably presented to the nutritionist as the preferred user for creating and/or editing a food shelf 160. This screen display comprises an input field 162 for receiving the input of the name for new food shelf 160. Through further input fields 164 the food category and/or a food sub-category for the new food shelf 160 may be defined or input. In a food product presenting field 166 but screen display presents a list of graded food products, where the grading or rating or classification or rank is indicated by colored squares next to the name of the food product. The color code used for grading the food products is preferably similar to the color code (e.g. green/yellow/red) for grading the food ingredients as shown in figure 4. Preferably, the food product presenting field presents all graded food products achieved from the food database 100 and which belong to the food category and/or the food subcategory defined or input in the input fields 164. By clicking to particular food products listed in the food product presenting field 166 the user can select these products for editing them to the food shelf 160.

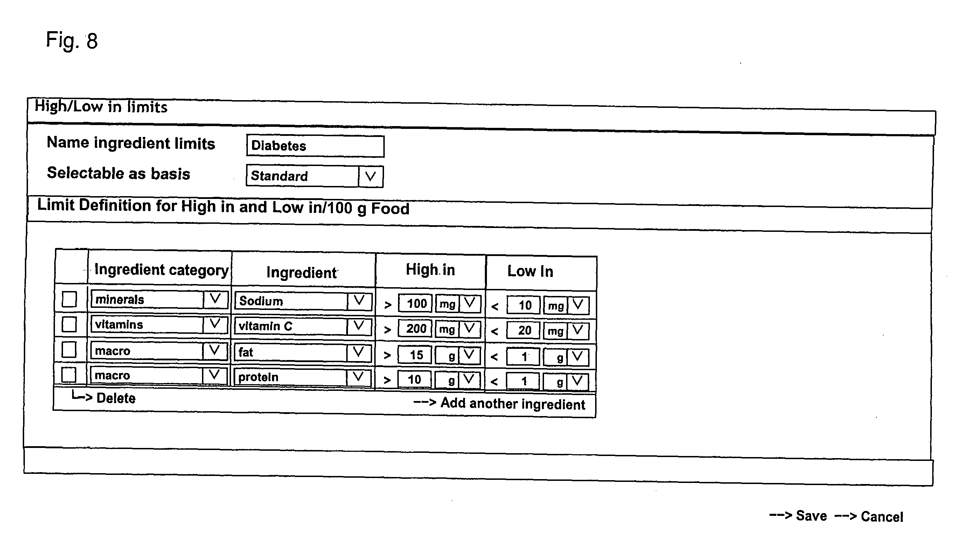

Further, the screen display or graphical user interface comprises filter definition input fields 168 for defining criteria according to which a filtering may be applied to the graded food products so as to achieve an automatic or semiautomatic preselection of the graded food products. According to this preferred embodiment at least one filter scheme is provided or stored that defines high and/or low limits for the content to some particular food ingredients in a specific (predetermined or predeterminable) amount (e.g. about 100 g) of a food product. Figure 8 shows a preferred screen display or graphical user interface for creating and/or editing such a filter scheme. Sometimes a nutrition plan should be based on the special therapy that requires the intake of a (relatively) high and/or low amount of a particular food ingredient. Such limits can be defined or input through the input interface as presented in figure 8 in an analogous manner as in the previously discussed input interfaces. In the example is shown in figure 7 the filter criteria a set to a high amount of vitamin C and a little amount of fat based on the limit values as defined in the filter scheme "standard". When applying this filter the number of food products presented in the list 166 can be reduced to those food products fulfilling the requirements defined through the filter criteria. After having selected a list of food products this list is assigned or associated to the food shelf and preferably saved on storage means preferably together with the database 100.

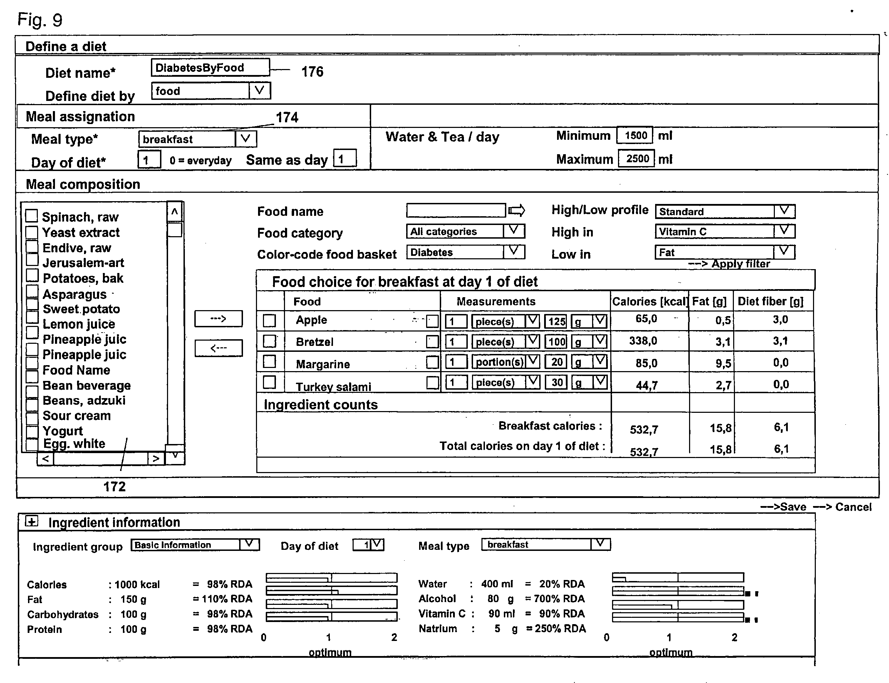

In another preferred embodiment the step S5 of assigning or associating the graded food products to the at least one patient comprises composing a well-defined diet 170 and assigning the well-defined diet to the at least one patient. Figure 9 shows a preferred screen display or graphical user interface for composing such a well-defined diet 170. In this well-defined diet 170 preferably a detailed list of food products for a particular meal (e.g. the breakfast, lunch or dinner) is defined. Similar to the previously shown screen displays a list of graded food products (preferably having a specific (predetermined or predeterminable) grading or rating or classification or rank) is presented in food product presenting field 172, from which they used to, preferably the nutritionist, chooses particular food products for composing a particular new, the type of which is defined in a meal type input field 174. Through this procedure a plurality of meals can be defined and assigned to the well-defined product 170 which is preferably saved on storage means preferably together with the food database 100 and with the name defined through a diet name input field 176. This well-defined diet 170 is assigned or associated to at least one patient and/or a group of patients so that this at least one patient and/or group of patients has access to the prescribed diet. Assigning the food shelf 160 and/or the well-defined diet 170 to at least one patient preferably means that the at least one patient has access to the data in the sense that he is able to read the data and follow the prescribed instructions. This access preferably is established through a computer interface (such as a graphical user interface) and/or a print out of the nutrition plan prescriptions. When using a (mono-, bi- and/or multidirektional) computer network a nutritionist preferably presents the food shelf 160 and/or the well-defined diet 170 in the computer network in such a manner that a patient using a remote computer has access to this data and can read the nutrition plan prescriptions. Alternatively a nutritionist hands out a printed version of the food shelf 160 and/or the well-defined diet 170 to the at least one patient. In this case it is sufficient to store the assigned food shelf 160 and/or well-defined diet 170 locally at the nutritionist's computer.

Secondly, addressing the second alternative of assigning the food basket 110 to at least one patient and/or a group of patients in step S5, the assigned food-baskets 110 preferably is applied to the food database 100 in step S4' at the patient's side analogous to the embodiment shown in figure 2. The step S5' of assigning the food basket 110 to the at least one patient preferably comprises the signing the food-baskets 110 to at least one diet program scheme 180 and assigning or associating the diet program scheme 180 to the at least one patient.

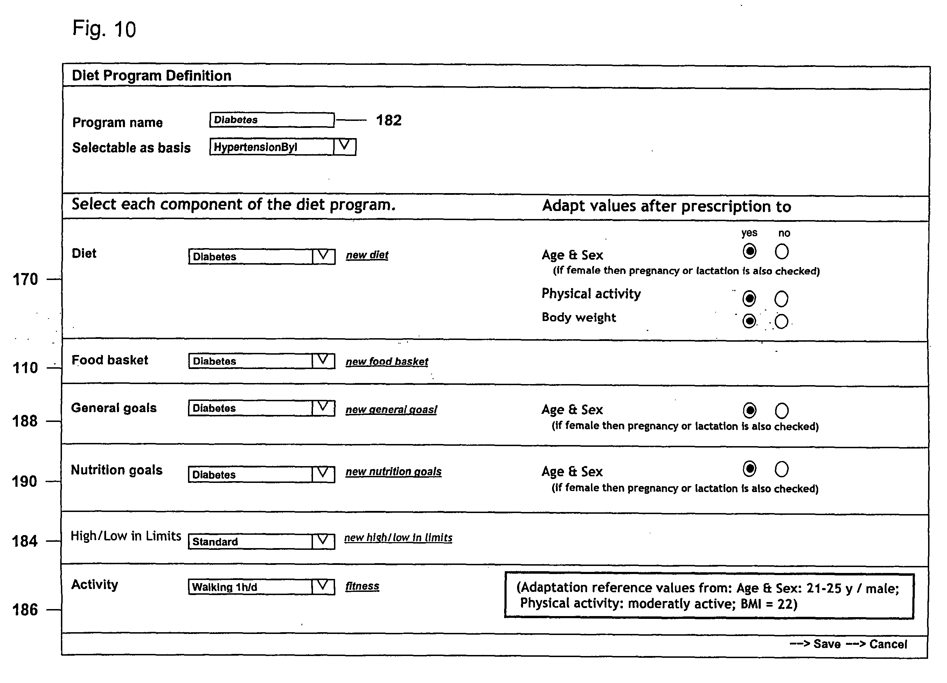

Figure 10 shows an exemplary screen display or a graphical user interface of an input interface for composing a diet program scheme the name of which is preferably inputted via a program name input fields 182. Moreover, a series of further input fields are presented for assigning or associating individual elements to the diet program scheme. Preferably these elements comprise the well-defined diet 170, which is preferably composed as described before, the food-baskets 110, which is assigned to the at least one patient together with the diet program scheme 180, a filter scheme 184 as described in connection with figure 8, physical activity prescriptions 186, general goals 188 and/or nutrition goals 190.

When assigning this diet program scheme to at least one patient, the method according to another preferred embodiment of the present invention comprises a step of receiving personal data regarding the at least one patient. These personal data preferably comprise age and/or sex of the at least one patient. It is even more preferred that a reference value adaptation scheme is provided for adapting standard reference values such as those defined in the food-baskets 110 to the personal data of the particular patient. While the standard reference values are preferably defined for a standard male patient with an age between 21 and 25, a reference value adaptation scheme allows to adapt these reference values to the individual requirements for the particular patient depending on its personal data preferably comprising its age and/or sex.

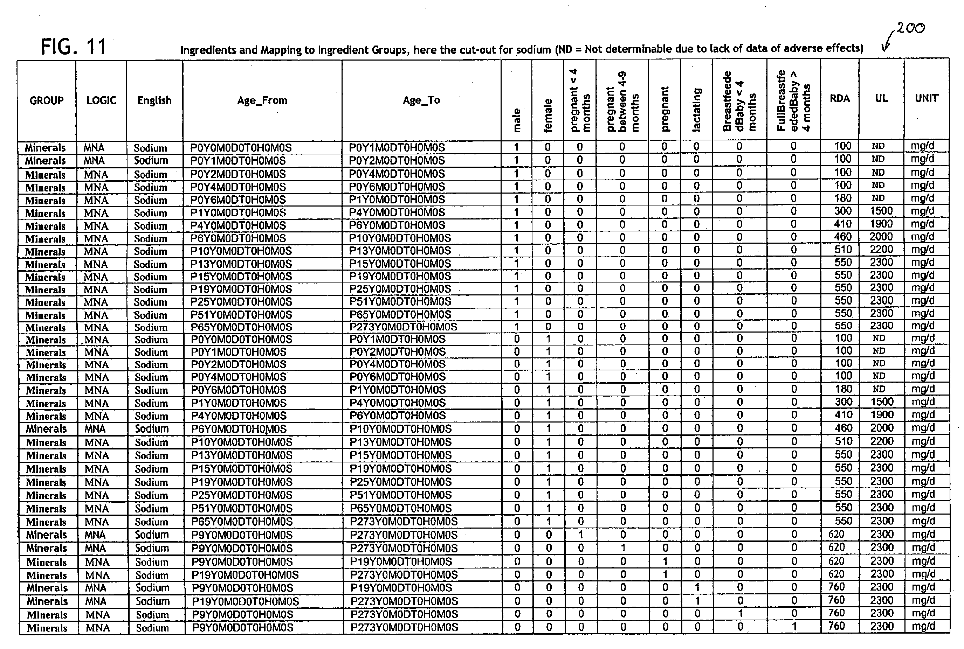

Figure 11 shows an exemplary excerpt from a table 200 which forms at least part of a preferred basis for the reference value adaptation scheme, wherein this exemplary table shows the recommended daily allowance (RDA) and the upper intake level or the intake limit (UL) for sodium for a plurality of particular personal status conditions such as sex, age, pregnancy, etc.. This exemplary table includes ingredients and mapping to ingredient groups (in particular the cut-out sodium (ND stands for "not determinable" particularly due to lack of data of adverse effects) and in particular shows absolute values for these reference values, wherein in another preferred embodiment the reference value adaptation scheme defines the ratio of the particular reference value for the particular personal status condition of the at least one patient compared to a standard patient.

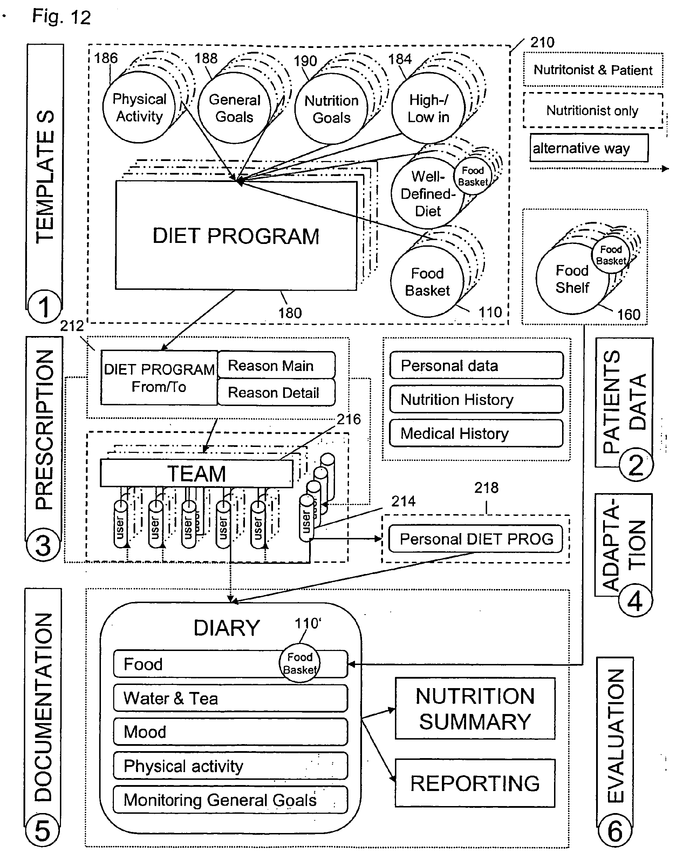

Figure 12 shows a block diagram of another preferred embodiment of the present invention. A diet program database 210 comprises a plurality of diet program schemes 180. The diet program database 210 is preferably stored in storage means where it is accessible for at least one nutritionist or doctor (as a preferred user) who draws up or establishes or generates nutrition plans. The diet program schemes 180 are preferably stored as templates and preferably serve as basis for drawing up a nutrition plan for at least one patient or a group of patients. Preferably each of diet program schemes 180 is composed of a plurality of diet program elements. These diet program elements comprise physical activity prescription schemes 186 and/or general goals schemes 188 and/or nutrition goal schemes 190 and/or filter schemes 184 and/or food ingredient grading schemes (food baskets) 110 and/or well-defined diet schemes. Preferably, a plurality of these schemes is provided as a template being part of the diet program database 210.

Preferably at least one of the predefined diet program schemes 180 is assigned or associated to a nutrition plan 212 for at least one patient 214 or a group of patients 216. The at least one patient 214 may be an individual patient or he may alternatively be a member of a team 216. In a preferred embodiment of the present invention a data structure comprises a patient database defining groups and/or teams of patients and their member as well as individual patients.

The nutrition plan 212 defines or comprises an assigned diet program that is preferably prescribed for a certain period of time. After assigning the diet program to the at least one patient it is preferably at least partly adapted or modified to the (more or less) precise personal requirements of the patient, i.e. the patient's data, for achieving a personal diet or nutrition program 218. Finally, the prescriptions of the nutrition plan and in particular the prescription defined in the personal diet program regarding the nutrition as well as physical activity are presented to the at least one patient. In particular, the at least one food basket 110' which is assigned to the patient as part of the diet program is apply to the food database 100 and/or a food shelf 160, which is preferably a pre-selected part of the food database 100, for achieving a grading or rating or classification or rank for a plurality of food products. The graded food products are presented to the patient and/or to the user (such as the nutritionist) together with the respective grading or rating or classification or rank of the food products for enabling the patient to chose suitable food products when composing his meals.

Finally, the patient preferably inputs his choices for his meals including food products and beverages during the whole term of the prescribed diet. From time to time the patient preferably receives a reporting that compares the originally aimed goals with the actually achieved results. The aimed goals preferably comprise general goals and/or nutrition goals according to preferred embodiments of the present invention.

The general goals and the nutritional goals preferably comprise high and/or low limits and/or preferred values for one or more of the parameters indicated in the following lists, respectively. Particularly, in the steps of receiving a reference value input according to preferred embodiments high and/or low limits and/or preferred values for one or more of the parameters listed in the following are received. One or more of these parameter values are preferably defined in respective goal schemes and more preferably stored in respective databases.

| **General Goal Group Name** | **General Goal Name** |
|---|---|
| Body masses | Body weight |
| | Body height |
| | Body-Mass-Index |
| | Waist2Hipp |
| | BodyImpedanz |
| | Systoloc blood pressure |
| | Diastolic blood pressure |
| | Heart rate |
| | Breathing rate |
| | Temperature (oral) |
| | Stool frequecy/d |
| Hematology | Iron binding capacity |
| | Erythrocytes |
| | Ferritin |
| | Basophile Granulocytes |
| | Eosinophile Granulocytes |
| | Neutrophile Granulocytes |
| | Hematokrit |
| | Hemoglobin |
| | Haptoglobin |
| | Basophile Leukocytes |
| | Eosinophile Leukocytes |
| | Segmented neutrophiles leukocytes |
| | Non-segmented neutrophiles leukocytes |
| | Leukocytes |
| | B-lymphocytes |
| | Lymph Lymphocytes |
| | T-helper cell lymphocytes |
| | T-lymphocytes |
| | T-suppressor cell lymphocytes |
| | Lymphocytes |
| | MCHC |
| | MCH |
| | MCV |
| | Monocytes |
| | Reticulocytes |
| | Platelets |
| | Transferrin |
| Inflammation | ESR |
| | ESR2 |
| | CRP |
| Electrolytes and Elements | Chloride |
| | Ceruloplasmin |
| | Iron in serum |
| | Potassium in serum |
| | Calcium in serum |
| | Total calcium in serum |
| | Ionized calcium in serum |
| | Copper in serum |
| | Magnesium in serum |
| | Sodium in serum |
| | Phosphate in serum |
| | Chloride in Urine |
| | Potassium in Urine |
| | Total calcium in urine |
| | Copper in urine |
| | Sodium in urine |
| Kidney & Urine | Albumin |
| | Erythrocytes (red blood cells) |
| | Uric acid |
| | Urea (only urea nitrogen) |
| | Creatinine clearance |
| | Creatinine |
| | Leucocytes |
| | Osmolality |
| | pH |
| | Protein |
| | Renin |
| | Specific Weight |
| Enzymes | Alkaline phosphatase (AP) |
| | Acid Phosphatase |
| | Alkaline PhosphateasBone |
| | AcetylcholinesteraseHe |
| | Weight CK-MB |
| | CPK |
| | Gamma Glutamyl transferase |
| | Glutamate Dehydrogenase |
| | AST |
| | ALT |
| | LDH-Isoenzyme 1 |
| | Infarct CK-MB |
| | LAP |
| | LDH |
| Liver & gall | Ammonia |
| | Direct bilirubin |
| | Total bilirubin |
| Liquor | Total protein |
| | Glucose |
| | Lactate |
| | Leukocytes |
| | IgG |
| | pH |
| | Specific weight |
| | Cells |
| Protein | Albumin |
| | Alpha1 anti-trypsin |
| | Alpha1 globulin |
| | Alpha2 globulin |
| | Beta2 micro globulin |
| | Beta Globulin |
| | Electro-phoresis |
| | Electro-phoresis alpha1 globulin |
| | Electro-phoresis alpha2 globulin |
| | Electro-phoresis beta globulin |
| | Electro-phoresis gamma globulin |
| | Gamma globulin |
| | Total protein |
| | Immuno-globulin A |
| | Immuno-globulin G |
| | Immuno-globulin M |
| Glucose Metabolism | Non-fasting blood glucose |
| | Fasting blood glucose |
| | Fasting c-peptide |
| | Urine glucose |
| | HbA1c |
| | Fasting insulin |
| Fat Metabolism | Total cholesterol |
| | HDL cholesterol |
| | LDL cholesterol |
| | LDL-/HDL-Quotient |
| | VLDL |
| | Triglyceride |
| Hormone | ACTH |
| | Epinephrine |
| | Aldosterone |
| | DHEAS |
| | 1,25 Dihydrocholecalciferol |
| | Free cortisol |
| | Free thyroxine (T4) |
| | 25-Hydroxy Vitamin D |
| | Cortisol |
| | Meta-nephrine |
| | Estradiol |
| | Prolactin |
| | HGH |
| | Testosterone |
| | Thyroxin (T4) |
| | Triiod-thyronine (T3) |
| | TSH |
| Stomach & pancreas | Chymo-trypsin |
| | Elastase 1 |
| | Gastrin |
| | Lipase |
| | Pancreatic Amylase |
| | Stool fat |
| Blood Type/ | Blood group |
| | Rhesus factor |
| | Rhesus formula |
| Coagulation | Anti-thrombin III |
| | PPT |
| | Bleeding time |
| | Fibrinogen |
| | INR |
| | Prothrombin time |
| | Thrombin-time |
| Blood Gas Analysis | Base excess |
| | Lactate |
| | 02 saturation |
| | PaCO2 |
| | PaO2 |
| | pH |
| | Bicarbonate |

| **Ingredient Category** | **Ingredient** |
|---|---|
| Macro Nutrient | Energy (kilo calories) |
| | Energy (kilo joule) |
| | Water_Total |
| | Water_Beverages |
| | Water_solid food |
| | Water_oxidation |
| | Protein |
| | Fat |
| | Carbohydrates, resorbable |
| | Fibres |
| | Mineral substances (crude ashes) |
| | Alcohol (ethyl alcohol) |
| Vitamins | Vitamin A - equivalent to retinol |
| | Vitamin A - retinol |
| | Vitamin A - beta-carotine |
| | Vitamin D - calciferole |
| | Vitamin E - equivalent to tocopherol |
| | Vitamin E - alpha tocopherol |
| | Vitamin K phyllochinone |
| | Vitamin B1 - thiamine |
| | Vitamin B2 - riboflavin |
| | Vitamin B3 - niacin, nicotinic acid |
| | Vitamin B5 - pantothenic acid |
| | Vitamin B6 - pyridoxine |
| | Vitamin B9 - equivalent to free folic acid |
| | Vitamin B9 - total folic acid |
| | Vitamin B9- free folic acid |
| | Vitamin B12-cobalamin |
| | Vitamin C - ascorbic acid |
| Minerals | Sodium |
| | Potassium |
| | Calcium |
| | Magnesium |
| | Phosphor |
| Trace elements | Iron |
| | Zinc |
| | Copper |
| | Manganese |
| Carbohydrates | Total sugar alcohols |
| | Glucose (dextrose) |
| | Fructose (fruit sugar) |
| | Galactose |
| | Saccharose (beet sugar) |
| | Maltose (malt sugar) |
| | Lactose (milk sugar) |
| | Starch |
| Aminoacids (Protein) | Iso-leucine |
| | Leucine |
| | Lysine |
| | Methionine |
| | Cysteine |
| | Phenylalanine |
| | Tyrosine |
| | Threonine |
| | Tryptophane |
| | Valine |
| | Arginine |
| | Histidine |
| | Alanine |
| | Asparaginic acid |
| | Glutamic acid |
| | Glycine |
| | Proline |
| | Serine |
| Fats_Saturated | Butyric acid/butanoic acid |
| | Hexanoic acid/caproic acid |
| | Octanoic acid/caprylic acid |
| | Decanoic acid/capric acid |
| | Dodecanoic acid/lauric acid |
| | Tetradecanoic acid/myristic acid |
| | Pentadecanoic acid |
| | Hexadecanoic acid/palmitic acid |
| | Heptadecanoic acid |
| | Octadecanoic acid/stearic acid |
| | Eicosanoic acid/arachidic acid |
| | Decosanic acid |
| | Tetracosanic acid |
| | Saturated fatty acids |
| Fats_Simple unsaturated | Tetradecenoic acid |
| | Pentadecenoic acid |
| | Hexadecenoic acid/palmitoleic acid |
| | Heptadecenoic acid |
| | Octadecenoic acid/oleic acid |
| | Eicosenoic acid |
| | Decosenoic acid/erucic acid |
| | Teretracosanoic acid |
| | Simple unsaturated fatty acids |
| Fats_Multiple unsaturated | Omega3_Octadecadienoic acid/linoleic acid |
| | Omega6_Octadecatrienoic acid/linolenic acid |
| | Octadecatetraenoic acid/stearidonic acid |
| | Eicosadienoic acid |
| | Eicosatrienoic acid |
| | Omega6_Eicosane-tetraenoic acid/arachidonic acid |
| | Omega3_Eicosapentaenoic acid |
| | Docosapentaenoic acid |
| | Omega3_Docosahexaenoic acid |
| Fats_Omega3_Multiple unsaturated | Omega3 fatty acids = sum (F183+F205+F226) |
| Fats_Omega6_Multiple unsaturated | Omega6 fatty acids = sum (F182+F204) |
| Fats_Multiple unsaturated | Multiple unsaturated fatty acids |
| Fats_Cholesterol | Cholesterol |

Further, the types of food ingredients and/or food ingredient categories defined and/or used in connection with the present invention are preferably selected from this table.

The invention can be implemented in digital electronic circuitry, or in computer hardware, firmware, software, or in combinations of them. The invention can be implemented as a computer program product, i.e., a computer program tangibly embodied in an information carrier, e.g., in a machine-readable storage device or in a propagated signal, for execution by, or to control the operation of, data processing apparatus, e.g., a programmable processor, a computer, or multiple computers. A computer program can be written in any form of programming language, including compiled or interpreted languages, and it can be deployed in any form, including as a stand-alone program or as a module, component, subroutine, or other unit suitable for use in a computing environment. A computer program can be deployed to be executed on one computer or on multiple computers at one site or distributed across multiple sites and interconnected by a communication network.

Method steps of the invention can be performed by one or more programmable processors executing a computer program to perform functions of the invention by operating on input data and generating output. Method steps can also be performed by, and apparatus of the invention can be implemented as, special purpose logic circuitry, e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory (ROM) or a random access memory (RAM) or both. The basic elements of a computer are a processor for executing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. Information carriers suitable for embodying computer program instructions and data include all forms of non-volatile memory, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in special purpose logic circuitry.

The invention can be implemented in a computing system that includes a back-end component, e.g., as a data server, or that includes a middleware component, e.g., an application server, or that includes a front-end component, e.g., a client computer having a graphical user interface or a Web browser through which a user can interact with an implementation of the invention, or any combination of such back-end, middleware, or front-end components. The components of the system can be interconnected by any form or medium of digital data communication, e.g., a communication network. Examples of (mono-, bi- or multi-directional) communication networks include a local area network ("LAN"), a wireless local area network (WLAN) and a wide area network ("WAN"), e.g., the Internet.

The computing system can include clients and servers. A client and server are generally remote from each other and typically interact through a communication network. The relationship of client and server arises by virtue of computer programs running on the respective computers and having a client-server relationship to each other.

### List of Reference Numerals

- 20: computing environment
- 22: processing unit
- 24: system memory
- 26: system bus
- 28: random access memory (RAM)
- 30: read only memory (ROM)
- 32: hard disk drive
- 34: external disk drive
- 36: removable disk
- 38: hard disk interface
- 40: external disk interface
- 42: external disk
- 44: application program
- 46: program data
- 48: keyboard
- 50: mouse
- 52: serial port interface
- 54: parallel port interface
- 56: printer
- 58: monitor
- 60: video input/output
- 62: remote computer
- 64: local area network (LAN)
- 66: wide area network (WAN)
- 68: network I/O
- 70: modem
- 100: food database
- 102: table defining a food database
- 104: food product column
- 106: food ingredient columns
- 110, 110': food ingredient grading scheme
- 112: table
- 114: food ingredient column
- 116: grading column
- 118: reference column
- 120: weighting column
- 122, 122', 124: graded food products
- 130: menu
- 132: list of food ingredient grading schemes
- 134: input
- 136: receiving a food ingredient
- 138: receiving a weighting factor
- 140: receiving grading intervals
- 142: receiving a reference value
- 144: food basket selection fields
- 146: name input field
- 148: "combine"-button
- 150: list of ingredient gradings
- 152: allergic selection column
- 154: RDA column
- 156: UL column
- 158: branching
- 160: food shelf
- 162, 162: input fields
- 166: food product presenting field
- 168: filter definition input fields
- 170: well-defined diet
- 172: food product presenting field
- 174: meal type input field
- 176: diet name input field
- 180: diet program scheme
- 182: program name input field
- 184: filter scheme
- 186: physical activity prescriptions
- 188: general goals
- 190: nutrition goals
- 200: table for reference value adaptation scheme
- 210: diet program database
- 212: nutrition plan
- 214: patient
- 216: group of patients, team
- 218: personal diet program

## Claims

1. Computer-based method of drawing up a nutrition plan for at least one patient and/or a group of patients by a user comprising:
- a step (S1) of providing a food database (100) defining the composition of a plurality of food products composed of at least part of its food ingredients;
- a step (S2) of receiving an ingredient grading input by the user for establishing at least one grading for at least one food ingredient;
- a step (S3) of assigning the at least one grading to a food ingredient grading scheme (110);
- a step (S4, S4') of applying the food ingredient grading scheme (110) to at least part of the food database (100) for achieving a grading for at least part of the food products; and
- a step (S5, S5') of assigning the food ingredient grading scheme (110) and/or at least part of the graded food products (122) to the at least one patient and/or the group of patients.

2. Method according to claim 1, wherein the step (S5) of assigning at least part of the graded food products to the at least one patient and/or the group of patients comprises the steps of
- presenting a plurality of graded food products (122) together with the grading of the food products;
- receiving a food product selection input by the user for achieving a selection of graded food products (122'); and
- assigning the selection of graded food products (122') to the at least one patient.

3. Method according to claim 1 or 2, wherein the step (S5, S5') of assigning the food ingredient grading scheme (110) and/or at least part of the graded food products (122) to the at least one patient and/or the group of patients comprises
- composing at least one diet program scheme (180) comprising the food ingredient grading scheme (110) and/or at least part of the graded food products (122); and
- assigning the diet program scheme (180) to the at least one patient and/or the group of patients.

4. Method according to claim 3, further comprising the steps of
- receiving a general goal reference value input for establishing at least one general goal scheme (188) defining at least one reference value for low and/or high limits for at least one of the group comprising body weight, body mass index, blood pressure, heart rate, and total cholesterol; and/or
- receiving a nutrition goal reference value input for establishing at least one nutrition goal scheme (190) defining at least one reference value for low and/or high limits for the intake/day for at least one food ingredient; and/or
- receiving physical activity prescription input for establishing at least one set of physical activity prescriptions (186); and
where the method further comprises
- assigning the at least one general goal scheme (188) and/or the at least one nutrition goal scheme (190) and/or at least one set of physical activity prescriptions (186) to the at least one diet program scheme (180).

5. Method according to anyone of the preceding claims, wherein the step (S2) of receiving an ingredient grading input comprises
- a step (136) of receiving an ingredient selection input by the user defining at least one ingredient to be graded;
- a step (142) of receiving a grading reference value input for achieving at least one ingredient grading reference value, which the amount for the selected food ingredient should be compared to;
- a step (140) receiving a grading interval input by the user defining a plurality of grading intervals each of which defines a percentage interval relative to the reference value; and
- a step of assigning a grade to each of the grading intervals.

6. Method according to claim 4 or 5, further comprising a step of providing at least one standard reference value database defining a standard reference value for at least one type of reference values selected from the reference values for the general goals (188) and/or the nutrition goals (190) and/or the food ingredient grading,
wherein the steps of receiving a reference value input comprise the steps of
- receiving a reference selection input for defining and/or selecting at least one reference value type; and
- retrieving the standard reference value for the defined reference value type from the at least one standard reference value database.

7. Method according to anyone of the claims 4 to 6, further comprising
- a step of providing a reference value adaptation scheme (200) that defines for a plurality of different personal status conditions comprising age and/or sex of a patient a ratio for at least one reference value relative to the standard reference value defined for standard status conditions;
- a step of receiving an input by the user for defining personal status data of the at least one patient, where the personal status data comprise age and/or sex of the at least one patient; and
- a step of applying the reference value adaptation scheme to the standard reference values depending on the personal data for achieving personal reference values.

8. Method according to anyone of the preceding claims, wherein the step (S2) of receiving an ingredient grading input comprises a step (138) of receiving a weighting input by the user defining a weighting factor for the at least one grading of the at least one food ingredient and where the grading of the at least one food product is achieved by considering the grading for at least part of its ingredients with a relative weight defined by the respective weighting factor.

9. Method according to anyone of the preceding claims and claim 2, further comprising the steps of
- receiving at least one high and/or low ingredient limit input by the user for defining at least one high and/or low limit for at least one food ingredient;
- assigning the at least one received ingredient limit to at least one food ingredient filter scheme (184); and
- applying the food ingredient filter scheme (184) to at least part of the food database (100) for achieving a filtering of at least part of the food products, where the filtering distinguishes between allowable and non-allowable food products; and
wherein in the step of presenting a plurality of graded food products only allowable food products are presented.

10. Method according to anyone of the preceding claims and claim 3 or 4, further comprising after assigning the diet program scheme to the at least one patient and/or the group of patients receiving an adaptation input for adapting at least one of the food ingredient grading scheme, the general goals, the nutrition goals, the filter scheme and the diet to the at least one patient and/or the group of patients.

11. Computer-based method of drawing up a nutrition plan for at least one patient and/or a group of patients comprising
- presenting at least one food product;
- retrieving at least one ingredient of the food product from a food database (100);
- retrieving at least one grading for the at least one ingredient of the food product from a food ingredient grading scheme (110') assigned to the at least one patient and/or the group of patients;
- grading (S4') the food product depending on the at least one grading of the at least one of its ingredients; and
- presenting the grading of the food product (124) together with the food product.

12. Method according to claim 11, wherein retrieving at least one ingredient comprises retrieving a plurality of ingredients of the at least one food product;
wherein determining at least one grading for the at least one ingredient comprises
- determining at least one grading for at least part of the plurality of ingredients and
- determining [setting] a weighting factor of the at least one grading for each ingredient; and
where in the step of grading the at least one food product the grading for each ingredient of the food product is considered with a weight defined by the associated weighting factor.

13. Computer system for drawing up or generating a nutrition plan for at least one patient and/or a group of patients by a user comprising:
- food product composition storage means for storing at least one food database defining the composition of a plurality of food products composed of at least part of its food ingredients;
- ingredient grading input means for receiving an input by the user for establishing at least one grading for at least one food ingredient;
- grading scheme composition means for composing at least one food ingredient grading scheme by assigning the at least one grading for at least one food ingredient to the at least one food ingredient grading scheme;
- food product grading means for grading at least part of the food products defined in the at least one food database depending on the grading of at least one of its ingredients according at least one food ingredient grading scheme; and
- nutrition plan composition means for assigning the food ingredient grading scheme and/or at least part of the graded food products to a nutrition plan for the at least one patient and/or the group of patients.

14. Computer system for drawing up or generating a nutrition plan for at least one patient and/or a group of patients by a user comprising:
- food product composition storage means for storing at least one food database defining the composition of a plurality of food products composed of at least part of its food ingredients;
- food ingredient grading storage means for storing at least one food ingredient grading scheme assigned to the at least one patient and/or the group of patients, where the at least one food ingredient grading scheme defines at least one grading for at least one food ingredient;
- food product grading means for grading at least one food product defined in the at least one food database depending on the grading of at least one of its ingredients according to the at least one food ingredient grading scheme; and
- food product presenting means for presenting the at least one graded food product together with its grading.

15. Computer program product comprising program code, which when loaded in a computer system causes the system to perform step according to a method of one of the claims 1 to 12.

16. Computer-implemented data structure integrated as an element of a nutrition plan for at least one patient, and/or a group of patients comprising:
- at least one food database defining the composition of a plurality of food products composed of at least part of its food ingredients;
- at least one food ingredient grading scheme defining a grading for at least one food ingredient;
where a link is established from the at least one graded food ingredient to the respective ingredient defined in the at least one food database as a component of at least one food product such that this link results in a grading of the at least one food product.

17. Data structure according to claim 16 further comprising
- patient database for defining personal data for at least one patient or a group of patients;
- a diet program scheme database, that defines at least one diet program scheme; and
where the data structure further comprises
- a physical activity database comprising at least one physical activity prescription scheme (186) defining a prescription for physical activity as part of a nutrition plan; and/or
- a general goal database comprising at least one general goal scheme (188) defining at least one reference value for low and/or high limits for at least one of the group comprising body weight, body mass index, blood pressure, heart rate, and total cholesterol; and/or
- a nutrition goal database comprising at least one nutrition goal scheme (190) defining at least one reference value for low and/or high limits for the intake/day for at least one food ingredient; and/or
- a filter scheme database comprising at least one ingredient filter scheme (184) defining at least one high and/or low limit for at least one food ingredient; and/or
- a diet scheme database comprising at least one diet scheme defining for at least one meal its composition of at graded food products;
where each diet program scheme defines at least one data link that assigns the at least one food ingredient grading scheme and/or physical activity scheme and/or general goal scheme and/or nutrition goal scheme and/or filter scheme and/or diet scheme to a nutrition plan for at least one patient and/or a group of patients.

18. Data structure according to claim 16 or 17, where the food ingredient grading scheme comprises reference values relative to which the food ingredients are graded, and where the data structure further comprises
- a personal database for defining personal data regarding the at least one patient; and
- a reference value database comprising at least one reference value data scheme defining reference values depending on the personal data for at least the nutrition goal scheme and/or the general goal scheme and/or the food ingredient grading scheme.
